# EUROPÄISCHE PATENTANMELDUNG

(11) **EP 4 000 419 A1**
(43) Veröffentlichungstag der Anmeldung: **25.05.2022**
(21) Anmeldenummer: 20209177.3
(22) Anmeldetag: 23.11.2020
(51) Int. Cl.: A23L 27/30, C07H 1/06

(54) **TROCKNUNG VON ALLULOSEKRISTALLEN**

(71) Anmelder: Savanna Ingredients GmbH, 50189 Elsdorf (DE)
(72) Erfinder: BONGERS, Ulrich, 50169 Kerpen (DE); JUNKLEWITZ, Anna, 42119 Wuppertal (DE); MOHR, Stephan, 53879 Euskirchen (DE); BUTZ, Steffen, 52372 Kreuzau (DE); KOCH, Timo, 50189 Elsdorf (DE); BOGNAR, MIchael, 41540 Dormagen (DE)
(74) Vertreter: Fabry, Bernd

(57) **Zusammenfassung**

Vorgeschlagen wird ein Verfahren zur Trocknung von Allulosekristallen umfassend oder bestehend aus folgenden Schritten:
(a) Bereitstellung von nicht-getrockneten Allulosekristallen,
(b) Temperaturbehandlung der Kristalle gemäß Schritt (a) bei einer Temperatur im Bereich von etwa 25 bis etwa 70 °C in einer Trocknungsanlage, wobei die Temperaturbehandlung
(b1) bei Atmosphärendruck und einer Verweilzeit im Bereich von etwa 5 Minuten bis etwa 5 Stunden erfolgt,
oder
(b2) unter vermindertem Druck und konstanter Temperatur und einer Verweilzeit von etwa 30 min bis 5 Stunden erfolgt,
und
(c) Konditionierung des in Schritt (b1) oder (b2) gewonnenen Zwischenprodukts, wobei
(c1) die Konditionierung über einen Zeitraum von etwa 30 Minuten bis 7 Stunden bei einer Temperatur im Bereich etwa 40 bis etwa 70 °C erfolgt,
oder
(c2) die Konditionierung über einen Zeitraum von etwa 15 bis 90 Stunden mit Luft von einer relativen Feuchtigkeit von etwa 30 bis etwa 60% und auf einer Temperatur von etwa 25 bis etwa 40 °C erfolgt.

## Beschreibung

### GEBIET DER ERFINDUNG

Die Erfindung befindet sich auf dem Gebiet der Lebensmitteltechnologie und betrifft ein Verfahren zur Trocknung von Allulosekristallen.

### TECHNOLOGISCHER HINTERGRUND

Allulose (Psicose) ist ein kalorienarmer Zucker mit ähnlich süßem Geschmack von Zucker. Allulose ist einer von vielen verschiedenen Zuckern, die in der Natur in sehr geringen Mengen vorkommen. Allulose wurde ursprünglich aus Weizen identifiziert und wurde seitdem in bestimmten Früchten wie Jackfrucht, Feigen und Rosinen gefunden. Allulose ist natürlich in kleinen Mengen in einer Vielzahl von süßen Lebensmitteln wie Karamellsauce, Ahornsirup und braunem Zucker enthalten. Allulose wird vom Körper absorbiert, aber nicht metabolisiert, sodass sie nahezu kalorienfrei ist.

Aufgrund des wachsenden Interesses eines großen Teils der Bevölkerung an *"gesunder Ernährung"* und *"gesunder Lebensweise"* allgemein, hat Allulose als kalorienfreier Zucker ein großes Interesse in der Lebensmittelindustrie und in der wissenschaftlichen Community erweckt.

Allulose wird oft in Form von Kristallen kommerzialisiert.

### RELEVANTER STAND DER TECHNIK

Verfahren zur Herstellung von Allulose in kristalliner Form sind aus dem Stand der Technik bekannt. Beispielweise offenbart das Dokument EP 2 552 241 B1 (CJ CHEILJEDANG CORP) ein Verfahren zum Herstellen von D-Psicose, welches folgende Schritte umfasst: (a) Entfernen von Verunreinigungen aus einer D-Psicose Lösung, um eine gereinigte D-Psicose Lösung zu erhalten, (b) Konzentrieren der gereinigten D-Psicose Lösung bei einer Temperatur von 60 °C bis 70 °C und (c) Kristallisieren der D-Psicose aus der konzentrierten D-Psicose Lösung in einem übersättigten Zustand unter einer metastabilen Zone. Das Verfahren umfasst das Gewinnen der bei der Kristallisation erhaltenen D-Psicose-Kristalle, und Waschen der Kristalle mit entionisiertem Wasser und das Trocknen der Kristalle. Das Trocknen der Kristalle kann in einem Fließbetttrockner oder einem Vakuumtrockner durchgeführt werden.

Jedoch sind die Hersteller von Allulose in kristalliner Form mit dem Problem konfrontiert, dass die getrockneten Allulosekristalle, abhängig von der Trocknungsmethode, sehr schnell wieder Feuchtigkeit aufnehmen. Diese Hygroskopizität getrockneter Allulosekristalle verursacht nicht nur Schwierigkeiten in der Handhabe, sondern auch eine Reduzierung der Lagerstabilität des Produktes.

Es besteht daher im Gebiet der Lebensmitteltechnologie ein starkes Bedürfnis nach energieschonenden Verfahren, die eine verbesserte Trocknung von hochwertigen Allulosekristallen ermöglichen.

### AUFGABE DER ERFINDUNG

Die Aufgabe der vorliegenden Erfindung hat somit darin bestanden, ein Verfahren zur Trocknung von Allulosekristallen zur Verfügung zu stellen, die frei von den eingangs geschilderten Nachteilen sind. Insbesondere sollten die gewonnenen Allulosekristalle eine geringe Hygroskopizität aufweisen.

Eine zweite Aufgabe der vorliegenden Erfindung hat somit darin bestanden, ein Verfahren zur Trocknung von Allulosekristallen zur Verfügung zu stellen, so dass die erhaltenen Allulosekristalle rieselfähig, leicht wasserlöslich und geschmacklich einwandfrei sind.

### BESCHREIBUNG DER ERFINDUNG

Gegenstand der vorliegenden Erfindung ist ein Verfahren zur Trocknung von Allulosekristallen umfassend oder bestehend aus folgenden Schritten:
(a) Bereitstellung von nicht-getrockneten Allulosekristallen,
(b) Temperaturbehandlung der Kristalle gemäß Schritt (a) bei einer Temperatur im Bereich von etwa 25 bis etwa 70 °C in einer Trocknungsanlage, wobei die Temperaturbehandlung
   (b1) bei Atmosphärendruck und einer Verweilzeit im Bereich von etwa 5 Minuten bis etwa 5 Stunden erfolgt,
      oder
   (b2) unter vermindertem Druck und konstanter Temperatur und einer Verweilzeit von etwa 30 min bis 5 Stunden erfolgt,
   und
(c) Konditionierung des in Schritt (b1) oder (b2) gewonnenen Zwischenprodukts, wobei
   (c1) die Konditionierung über einen Zeitraum von etwa 30 Minuten bis 7 Stunden bei einer Temperatur im Bereich etwa 40 bis etwa 70 °C erfolgt,
      oder
   (c2) die Konditionierung über einen Zeitraum von etwa 15 bis 90 Stunden mit Luft von einer relativen Feuchtigkeit von etwa 30 bis etwa 60% und auf einer Temperatur von etwa 25 bis etwa 40 °C erfolgt.

Überraschenderweise wurde gefunden, dass Allulosekristalle, die mittels des erfindungsgemäßen Verfahrens getrocknet wurden, ein rieselfähiges Produkt darstellen, und eine erheblich geringere Hygroskopizität aufweisen als Allulosekristalle, die mittels der Standardbedingungen getrocknet wurden. Demzufolge zeichnen sich die erhaltenen Allulosekristalle durch eine erhebliche Lagerstabilität aus.

Ferner wurde überraschenderweise gefunden, dass Allulosekristalle, die mittels des erfindungsgemäßen Verfahrens getrocknet wurden, insbesondere leicht löslich und sensorisch einwandfrei sind.

Allulose, auch Psicose genannt, ist eine Ketohexose. Für die Zwecke der vorliegenden Erfindung wird Allulose vorzugsweise in Form des D-Enantiomers bereitgestellt, d.h. D-Allulose (CAS-Nr. 551-68-8). D-Allulose kann in Form von verschiedenen Anomeren wie z.B. α-D-Allulose und β-D-Allulose vorliegen.

Im Sinne der vorliegenden Erfindung ist der Begriff *"Temperaturbehandlung"* des Schrittes (b) auch als *"Trocknung"* bzw. *"Temperierung"* zu verstehen.

Im Sinne der vorliegenden Erfindung ist der Begriff *"Konditionierung"* auch als eine *"Nachkristallisation"* zu verstehen, die in Abwesenheit eines Lösungsmittels erfolgt. Das bedeutet, dass die in den Schritten (b1) oder (b2) gewonnenen Zwischenprodukte während des Schrittes (c) mit einem Lösungsmittel nicht in Kontakt gebracht werden.

Im Sinne der vorliegenden Erfindung ist das verwendete Verfahren zur Herstellung der nicht-getrockneten Allulosekristalle gemäß Schritt (a) für die erfolgreiche Durchführung des erfindungsgemäßen Trocknungsverfahrens nicht entscheidend.

Jedoch weisen die nicht-getrockneten Allulosekristalle gemäß Schritt (a) in einer bevorzugten Ausführungsform eine Reinheit größer 95 % auf, vorzugsweise größer als 98 %, und insbesondere bevorzugt im Bereich von 98,5 bis 99,5 % - jeweils bezogen auf die Gesamttrockenmasse.

In einer bevorzugten Ausführungsform des erfindungsgemäßen Trocknungsverfahrens liegt mindestens 10 Gew.-%, mindestens 20 Gew.-%, mindestens 30 Gew.-%, mindestens 40 Gew.-%, mindestens 50 Gew.-%, mindestens 60 Gew.-%, mindestens 70 Gew.-%, mindestens 80 Gew.-%, mindestens 90 Gew.-%, mindestens 95 Gew.-% oder mindestens 98 Gew.-%, mindestens 99 Gew.-% der Allulose in den nicht-getrockneten Allulosekristallen des Schritts (a) in Form von β-D-Psicopyranose vor - jeweils wieder bezogen auf die Gesamttrockenmasse.

Im Sinne der vorliegenden Erfindung werden die nicht-getrockneten Allulosekristalle gemäß Schritt (a) mittels Abtrennung, insbesondere mittels Zentrifugation oder Filtration, von der Mutterlauge aus einer konzentrierten Allulosesuspension gewonnen.

In einer bevorzugten Ausführungsform weisen die nicht-getrockneten Allulosekristalle gemäß Schritt (a) einen Gehalt an Wasser von maximal etwa 40 Gew.-%, vorzugsweise von maximal etwa 35 Gew.-%, vorzugsweise von maximal etwa 30 Gew.-%, vorzugsweise von maximal etwa 25 Gew.-%, vorzugsweise von maximal etwa 20 Gew.-%, vorzugsweise von maximal etwa 15 Gew.-%, vorzugsweise von maximal etwa 10 Gew.-%, vorzugsweise von maximal etwa 9 Gew.-%, vorzugsweise von maximal etwa 8 Gew.-%, vorzugsweise von maximal etwa 7 Gew.-%, vorzugsweise von maximal etwa 6 Gew.-%, vorzugsweise von maximal etwa 5 Gew.-%, vorzugsweise von maximal etwa 4 Gew.-%, vorzugsweise von maximal etwa 3 Gew.-%, vorzugsweise von maximal etwa 2 Gew.-%, vorzugsweise von maximal etwa 1 Gew.-%, und bevorzugt von maximal etwa 0,5 Gew.-% auf.

In einer weiteren bevorzugten Ausführungsform weisen die nicht-getrockneten Allulosekristalle gemäß Schritt (a) einen Gehalt an Wasser von etwa 10-0,3 Gew.-% auf, vorzugsweise von etwa 8-0,4 Gew.-%, und insbesondere bevorzugt von etwa 5-0,5 Gew.-%.

Wie bereits erwähnt, werden die nicht-getrockneten Allulosekristalle gemäß Schritt (a) einer Temperaturbehandlung in einer Trocknungsanlage unterworfen. Die Temperaturbehandlung gemäß Schritt (b) - sowohl in der Variante (b1) als auch in der Variante (b2) - erfolgt bei einer Temperatur im Bereich von etwa 25 bis etwa 70 °C.

In einer bevorzugten Ausführungsform erfolgt die Temperaturbehandlung des Schrittes (b) bei einer Temperatur im Bereich von etwa 30 bis etwa 65 °C und insbesondere bevorzugt im Bereich von etwa 30 bis etwa 60 °C.

Im Sinne der vorliegenden Erfindung kommen verschiedene Trocknungsanlagen für die Durchführung der Temperaturbehandlung gemäß Schritt (b) in Frage, beispielsweise Trommeltrockner, Bandtrockner, Schachttrockner, Konustrockner, Konvektionstrockner, Vibrationsfließbetttrockner, Wirbelschichttrockner, Rohrbündeltrockner, Dünnschichttrockner, Scheibentrockner, Vakuumtrockner, Kontakttrockner und Mikrowellentrockner. Diese Trocknungsanlagen sind für den Fachmann bestens bekannt und brauchen daher nicht näher erläutert werden.

Jedoch hat sich als besonders vorteilhaft die Durchführung der Temperaturbehandlung gemäß Schritt (b) in einer Trocknungsanlage ausgewählt aus der Gruppe bestehend aus Wirbelschichttrockner (kontinuierlich), Vakuumtrockner, Bandtrockner und Dünnschichttrockner, insbesondere Wirbelschichttrockner und Vakuumtrockner, erwiesen.

Erfindungsgemäß kann die Temperaturbehandlung entweder mittels der Variante (b1) oder mittels der Variante (b2) erfolgen.

Wie bereits erwähnt, erfolgt die Temperaturbehandlung gemäß (b1) bei Atmosphärendruck und einer Verweilzeit in der Trocknungsanlage im Bereich von etwa 5 Minuten bis etwa 5 Stunden.

In einer bevorzugten Ausführungsform erfolgt die Temperaturbehandlung gemäß (b1) bei konstanter Temperatur (die selbstverständlich in den in (b) definierten Bereich fällt).

In einer weiteren bevorzugten Ausführungsform weist die Trocknungsanlage in (b1) verschiedenen Heizzonen auf. Die verschiedenen Heizzonen sind die individuell steuerbar, so dass es möglich ist, ein beliebiges Temperaturprofil anzulegen.

Die Trocknungsanlage in (b1) weist in einer bevorzugtem Ausführungsform 2 bis 5 verschiedene Heizzonen auf. Jeder Heizzone weist eine unterschiedliche Temperatur auf, wobei die Temperaturen jeder Heizzone selbstverständlich in den in (b) definierten Bereich fallen; nicht-benachbarte Heizzonen können die gleiche Temperatur aufweisen. In einer bevorzugten Ausführungsform beträgt die Verweilzeit pro Heizzone der Trocknungsanlage etwa 1 bis etwa 8 Minuten, vorzugsweise etwa 3 bis etwa 8 Minuten, bevorzugt etwa 4 bis etwa 7 Minuten.

Es hat sich als energetisch besonders vorteilhaft herausgestellt, wenn die Trocknungsanlage im Schritt (b1) drei verschiedene Heizzonen aufweist. In einer bevorzugten Ausführungsform entspricht dem Temperaturprofil folgendes Schema:
T₁. Heizzone < T₂. Heizzone >T₃. Heizzone, WObei T₁. Heizzone ≥ T₃. Heizzone
   oder
T₁. Heizzone < T₂. Heizzone >T₃. Heizzone, WObei T₁. Heizzone ≤ T₃. Heizzone
In einer bevorzugten Ausführungsform sind die Temperaturen in den 3 verschiedenen Heizzonen wie folgend eingestellt:
- T₁. Heizzone liegt im Bereich von 30-45 °C, bevorzugt, 35-40 °C, und insbesondere bevorzugt 40 °C;
- T₂. Heizzone liegt im Bereich von 50-70 °C, bevorzugt, 55-65 °C, und insbesondere bevorzugt 60 °C;
- T₃. Heizzone liegt im Bereich von 25-35 °C, bevorzugt, 25-30 °C, und insbesondere bevorzugt 30 °C.
Wenn die Trocknungsanlage drei unterschiedlichen Heizzonen aufweist, beträgt die Verweilzeit pro Heizzone der Trocknungsanlage in einer bevorzugten Ausführungsform etwa 3 bis 8 Minuten, vorzugsweise 4 bis 6 Minuten, und insbesondere bevorzugt 4 Minuten.

Wie bereits erwähnt, erfolgt die Temperaturbehandlung gemäß (b2) unter vermindertem Druck und konstanter Temperatur (die selbstverständlich in den in (b) definierten Bereich fällt) und einer Verweilzeit in der Trocknungsanlage von etwa 30 min bis 5 Stunden.

In einer bevorzugten Ausführungsform wird die Temperaturbehandlung gemäß Schritt (b2) bei einem Druck von etwa 5 bis etwa 300 mbar durchgeführt, vorzugsweise von etwa 10 bis etwa 250 mbar und besonders bevorzugt bei einem Druck von etwa 20 bis etwa 200 mbar.

In einer bevorzugten Ausführungsform erfolgt die Temperaturbehandlung gemäß Schritt (b2) bei einer Temperatur im Bereich von etwa 25 bis etwa 45 °C, vorzugsweise von etwa 30 bis etwa 40 °C, und besonders bevorzugt bei etwa 30 bis etwa 35 °C.

In einer weiteren bevorzugten Ausführungsform beträgt die Verweilzeit in der Trocknungsanlage etwa 1 bis etwa 4 Stunden, vorzugsweise etwa 2 bis etwa 4 Stunden, und insbesondere bevorzugt etwa 3 Stunden.

In einer besonders bevorzugten Ausführungsform erfolgt die Temperaturbehandlung gemäß Schritt (b2) bei einem Druck von etwa 20 bis etwa 200 mbar und einer Temperatur von etwa 30 bis etwa 40 °C und einer Verweilzeit in der Trocknungsanlage von etwa 3 Stunden.

Der Wassergehalt des in Schritt (b1) oder (b2) gewonnenen Zwischenprodukts liegt im Bereich von etwa 0,1 bis etwa 0,5 Gew.-%, vorzugsweise von etwa 0,1 bis etwa 0,3 Gew.-%, bevorzugt von etwa 0,15 bis 0,25 Gew.-%.

Obwohl das in Schritt (b1) oder (b2) gewonnene Zwischenprodukt einen deutlich reduzierten Wassergehalt im Vergleich zu den nicht-getrockneten Allulosekristallen des Schrittes (a) aufweist, sind die Eigenschaften dieses Zwischenprodukt nicht zufriedenstellend, besonders im Hinblick auf die Rieselfähigkeit des Produktes.

Erfindungsgemäß erfolgt nach der Temperaturbehandlung eine Konditionierung des in Schritt (b1) oder (b2) gewonnenen Zwischenprodukts. Darunter versteht man, die in Schritt (b1) oder (b2) gewonnenen Zwischenprodukte über einen bestimmten Zeitraum bei einer bestimmten Temperatur bzw. in Kontakt mit einer Luft, die spezifische Eigenschaften im Hinblick auf die Wasserbeladung aufweist, zu lagern, so dass eine Art von *"Nachkristallisation"* erfolgt.

Im Sinne der vorliegenden Erfindung kommen verschiedene Vorrichtungen für die Durchführung der Konditionierung gemäß Schritt (c) - sowohl in der Variante (c1) als auch in der Variante (c2) - in Frage, beispielsweise Trommeltrockner, Bandtrockner, Schachttrockner, Konustrockner, Konvektionstrockner, Vibrationsfließbetttrockner, Wirbelschichttrockner, Rohrbündeltrockner, Dünnschichttrockner, Scheibentrockner, Vakuumtrockner, Big Bags, oder Förderanlage zur pneumatischen Förderung (z,B. in einem Silo). Diese Trocknungsanlagen sind für den Fachmann bestens bekannt und brauchen daher nicht näher erläutert werden.

In einer bevorzugten Ausführungsform erfolgt die Konditionierung gemäß Schritt (c) in einer Vorrichtung ausgewählt aus der Gruppe bestehend aus Trommeltrockner, Wirbelschichttrockner, Vakuumtrockner, Dünnschichttrockner oder einer Förderanlage zur pneumatischen Förderung (z,B. in einem Silo).

In Sinne der vorliegenden Erfindung erfordert die Durchführung der Konditionierung nicht notwendigerweise die Übertragung des Zwischenprodukts gemäß (b1) oder (b2) von einer ersten Vorrichtung, in der die Temperaturbehandlung erfolgte, zu einer zweiten Vorrichtung, in der die Konditionierung erfolgen soll. Es ist durchaus möglich, die Schritte (b) und (c) in ihrem jeweiligen Varianten in der gleichen Vorrichtung durchzuführen. So kann beispielweise eine Temperaturbehandlung gemäß (b2) in einem Vakuumtrockner, und die Konditionierung in dem gleichen Vakuumtrockner erfolgen.

In einer ersten Variante (c1) erfolgt die Konditionierung über einen Zeitraum von etwa 30 Minuten bis 7 Stunden bei einer Temperatur im Bereich etwa 40 bis etwa 70 °C.

In einer bevorzugten Ausführungsform erfolgt die Konditionierung gemäß (c1) bei einer Temperatur im Bereich etwa 50 bis etwa 70 °C, vorzugsweise im Bereich etwa 55 bis etwa 65 °C, insbesondere bevorzugt im Bereich etwa 58 bis etwa 62 °C.

In einer weiteren bevorzugten Ausführungsform erfolgt die Konditionierung gemäß (c1) über einen Zeitraum von etwa 30 Minuten bis 6 Stunden bei einer Temperatur > 50 °C.

In einer ganz besonderes bevorzugten Ausführungsform erfolgt die Konditionierung gemäß (c1) über einen Zeitraum von etwa 3 bis 6 Stunden bei einer Temperatur von etwa 60 °C.

In der Variante (c2) erfolgt die Konditionierung über einen Zeitraum von etwa 15 bis etwa 90 Stunden mit Luft von einer relativen Feuchtigkeit von etwa 30 bis etwa 60% und auf einer Temperatur von etwa 25 bis etwa 40 °C.

In einer bevorzugten Ausführungsform erfolgt die Konditionierung gemäß (c2) über einen Zeitraum von etwa 24 bis etwa 72 Stunden und Luft auf eine Temperatur von etwa 30 bis etwa 35 °C, vorzugsweise 30 °C.

In einer weiteren Ausführungsform erfolgt die Konditionierung gemäß (c2) mit Luft von einer relativen Feuchtigkeit von etwa 35 bis etwa 55 %, vorzugsweise von etwa 35 bis etwa 45 %, und besonders bevorzugt etwa 40 bis etwa 45 %.

In einer besonders bevorzugten Ausführungsform erfolgt die Konditionierung gemäß (c2) über einen Zeitraum von etwa 24 bis etwa 72 Stunden und mit Luft von einer relativen Feuchtigkeit von etwa 40 % und auf eine Temperatur von etwa 30 °C.

In einer weiteren bevorzugte Ausführungsform erfolgt die Konditionierung gemäß (c2) in einem Dünnschichttrockner, wobei das verteilte Zwischenprodukt aus der Temperaturbehandlung eine Schichtdicke < 10 mm aufweist.

Die getrockneten Allulosekristalle, die mittels des erfindungsgemäßen Verfahrens gewonnen werden, weisen einen Gehalt an Wasser von maximal 0,1 Gew.-%, vorzugsweise von maximal 0,01 Gew.-%, und besonders bevorzugt von maximal 0,001 Gew.-% auf.

Demzufolge ist auch ein Gegenstand der vorliegenden Erfindung Allulosekristalle, die einen Gehalt an Wasser von maximal 0,001 Gew.-% aufweisen, wobei die getrockneten Allulosekristalle mittels des oben geschilderten Verfahrens hergestellt werden.

Zusammenfassend lässt sich feststellen, dass das erfindungsgemäße Verfahren die Gewinnung von hochwertigen Allulosekristallen, die rieselfähig sind und durch eine ausgezeichnete Löslichkeit, geringe Hygroskopizität sowie durch ausgezeichnete sensorische Eigenschaften auszeichnen, ermöglicht.

### BEISPIELE

Die vorliegende Erfindung wird unter Bezugnahme auf die nachstehenden Beispiele leichter zu verstehen sein.

Jedoch dienen diese Beispiele lediglich zur Veranschaulichung der Erfindung und können nicht als einschränkend in Bezug auf den Schutzbereich der Erfindung ausgelegt werden.

### VERGLEICHSBEISPIEL 1 (V1)

Allulosekristalle wurden mittels Zentrifugation aus der Mutterlauge aus einer konzentrierten Allulose-Suspension (Gehalt an Allulose an der Gesamttrockensubstanz von 97 Gew.-%) gewonnen. Die abgetrennten Allulosekristalle wurden dann für 24 Stunden in einem Vakuumtrockenschrank bei 30 °C und einem Druck von 10 mbar getrocknet. Es wurde ein Produkt erhalten, welches eine Reinheit von 98% und einen Wassergehalt von 0,15 Gew.-% aufwies. Es konnte festgestellt werden, dass das Produkt stark hygroskopisch und nicht rieselfähig war.

### BEISPIEL 1 (ERFINDUNGSGEMÄSS)

Allulosekristalle wurden mittels Zentrifugation aus der Mutterlauge aus einer konzentrierten Allulose-Suspension (Gehalt an Allulose an der Gesamttrockensubstanz von 97 Gew.-%) gewonnen.
Die abgetrennten Allulosekristalle wurden einer Temperaturbehandlung in einem kontinuierlichen Wirbelschichttrockner unterworfen. Der Wirbelschichttrockner wies drei Heizzonen auf, wobei
T₁. Heizzone = 40 °C,
T₂. Heizzone = 60 °C, und
T₃. Heizzone = 30 °C.
Die Verweilzeit pro Heizzone des Wirbelschichttrockners betrug je 4 Minuten. Das gewonnene Zwischenprodukt wies einen Restwassergehalt von 0,2 Gew.-% auf, und war nicht rieselfähig.
Anschließend wurde dieses Zwischenprodukt einer Konditionierung in einer rotierenden Trockentrommel für 3 Stunden und bei einer Temperatur von 60 °C unterworfen.
Die so erhaltenen Allulosekristalle wiesen einen Gehalt an Wasser von maximal 0,07 Gew.-% auf. Die so erhaltenen Allulosekristalle stellten ein rieselfähiges Produkt dar.

### BEISPIEL 2 (ERFINDUNGSGEMÄSS)

Allulosekristalle wurden mittels Zentrifugation aus der Mutterlauge aus einer konzentrierten Allulose-Suspension (Gehalt an Allulose an der Gesamttrockensubstanz von 97 Gew.-%) gewonnen.
Die abgetrennten Allulosekristalle wurden einer Temperaturbehandlung in einem kontinuierlichen Wirbelschichttrockner für 30 Minuten bei einer Temperatur von 60 °C unterworfen. Das gewonnene Zwischenprodukt wies einen Restwassergehalt von 0,2 Gew.-% auf, und war nicht rieselfähig.
Anschließend wurde dieses Zwischenprodukt einer Konditionierung im kontinuierlichen Wirbelschichttrockner für 3 Stunden und bei einer Temperatur von 60 °C unterworfen.
Die so erhaltenen Allulosekristalle wiesen einen Gehalt an Wasser von maximal 0,08 Gew.-% auf. Die so erhaltenen Allulosekristalle stellten ein rieselfähiges Produkt dar.

### BEISPIEL 3 (ERFINDUNGSGEMÄSS)

Allulosekristalle wurden mittels Zentrifugation aus der Mutterlauge aus einer konzentrierten Allulose-Suspension (Gehalt an Allulose an der Gesamttrockensubstanz von 97 Gew.-%) gewonnen.
Die abgetrennten Allulosekristalle wurden einer Temperaturbehandlung in einem Vakuumtrockner für 3 Stunden bei einer Temperatur von 30 °C und einem Druck von 150 mbar unterworfen. Das gewonnene Zwischenprodukt wies einen Restwassergehalt von 0,2 Gew.-% auf, und war nicht rieselfähig.
Anschließend wurde dieses Zwischenprodukt einer Konditionierung in einem Dünnschichttrockner für 24 h mit Luft von 25 °C mit einer Wasserbeladung von 40% (entsprechend 8,0 g Wasser pro kg trockener Luft) unterworfen.
Die so erhaltenen Allulosekristalle wiesen einen Gehalt an Wasser von maximal 0,1 Gew.-% auf. Die so erhaltenen Allulosekristalle stellten ein rieselfähiges Produkt dar.

### BEISPIEL 4 (ERFINDUNGSGEMÄSS)

Allulosekristalle wurden mittels Zentrifugation aus der Mutterlauge aus einer konzentrierten Allulose-Suspension (Gehalt an Allulose an der Gesamttrockensubstanz von 97 Gew.-%) gewonnen.
Die abgetrennten Allulosekristalle wurden einer Temperaturbehandlung in einem Vakuumtrockner für 3 Stunden bei einer Temperatur von 30 °C und einem Druck von 150 mbar unterworfen. Das gewonnene Zwischenprodukt wies einen Restwassergehalt von 0,2 Gew.-% auf und war nicht rieselfähig.
Die nachfolgende Konditionierung erfolgte durch Umwälzung des Produkts mittels pneumatischer Förderung in einem Silo (4 Stunden bei 60 °C).
Die so erhaltenen Allulosekristalle wiesen einen Gehalt an Wasser von maximal 0,001 Gew.-% auf. Die so erhaltenen Allulosekristalle stellten ein rieselfähiges Produkt dar.

### EVALUIERUNG DER KRISTALLE

Die Löslichkeit in Wasser (g/Liter), die Zunahme des Wassergehaltes sowie die sensorischen Eigenschaften wurden nach Lagerung der erhaltenen Allulosekristalle in einem offenen Gefäß für 24 Stunden von einem Panel bestehend aus fünf erfahrenen und geschulteren Testern evaluiert (3 = ausgeprägt, 2 = vorhanden, 1 = nicht festzustellen). Die Ergebnisse sind in **Tabelle 1** zusammengefasst.

**Tabelle 1**

| Löslichkeit, Sensorik und Zunahme an Wassergehalt nach Lagerung der getrockneten Kristalle in einem offenen Gefäß für 24 Stunden | | | |
|---|---|---|---|
| **Beispiele** | **Löslichkeitsindex (ml)** | **Sensorik (Mittelwert)** | **Zunahme an Wassergehalt (%)** |
| **1** | < 0,1 | 1 | < 0,1 |
| **2** | < 0,1 | 1 | < 0,1 |
| **3** | < 0,1 | 1 | < 0,1 |
| **4** | < 0,1 | 1 | < 0,1 |
| **V1** | < 0,2 | 2 | > 0,2 |

Die experimentellen Daten zeigen, dass die mittels des erfindungsgemäßen Verfahrens getrockneten Allulosekristalle verbesserte Ergebnisse hinsichtlich Löslichkeit und Sensorik zeigen.

Darüber hinaus sind die mittels des erfindungsgemäßen Verfahrens getrockneten Allulosekristalle, diejenigen, bei denen die niedrigste Zunahme des Wassergehaltes nach einer Lagerung von 24 Stunden in einem offenen Gefäß erhalten wurde.

## Patentansprüche

1. Verfahren zur Trocknung von Allulosekristallen umfassend oder bestehend aus folgenden Schritten:
(a) Bereitstellung von nicht-getrockneten Allulosekristallen,
(b) Temperaturbehandlung der Kristalle gemäß Schritt (a) bei einer Temperatur im Bereich von etwa 25 bis etwa 70 °C in einer Trocknungsanlage, wobei die Temperaturbehandlung
(b1) bei Atmosphärendruck und einer Verweilzeit im Bereich von etwa 5 Minuten bis etwa 5 Stunden erfolgt,
oder
(b2) unter vermindertem Druck und konstanter Temperatur und einer Verweilzeit von etwa 30 min bis 5 Stunden erfolgt,
und
(c) Konditionierung des in Schritt (b1) oder (b2) gewonnenen Zwischenprodukts, wobei
(c1) die Konditionierung über einen Zeitraum von etwa 30 Minuten bis 7 Stunden bei einer Temperatur im Bereich etwa 40 bis etwa 70 °C erfolgt,
oder
(c2) die Konditionierung über einen Zeitraum von etwa 15 bis etwa 90 Stunden mit Luft von einer relativen Feuchtigkeit von etwa 30 bis etwa 60% und auf einer Temperatur von etwa 25 bis etwa 40 °C erfolgt.

2. Verfahren nach Anspruch 1, **dadurch gekennzeichnet, dass** die nicht-getrockneten Allulosekristalle gemäß Schritt (a) eine Reinheit größer als 95 % - bezogen auf die Gesamttrockenmasse - aufweisen.

3. Verfahren nach einem der vorhergehenden Ansprüche, **dadurch gekennzeichnet, dass** die nicht-getrockneten Allulosekristalle gemäß Schritt (a) einen Gehalt an Wasser von etwa 10-0,3 Gew.-% aufweisen.

4. Verfahren nach einem der vorhergehenden Ansprüche, **dadurch gekennzeichnet, dass** die Temperaturbehandlung des Schrittes (b) bei einer Temperatur im Bereich von etwa 30 bis etwa 65 °C erfolgt.

5. Verfahren nach einem der vorhergehenden Ansprüche, **dadurch gekennzeichnet, dass** die Temperaturbehandlung des Schrittes (b) in einer Trocknungsanlage ausgewählt aus der Gruppe bestehend aus Wirbelschichttrockner, Vakuumtrockner, Bandtrockner und Dünnschichttrockner.

6. Verfahren nach einem der vorhergehenden Ansprüche, **dadurch gekennzeichnet, dass** die Trocknungsanlage in (b1) 2 bis 5 verschiedene Heizzonen aufweist.

7. Verfahren nach Anspruch 6, **dadurch gekennzeichnet, dass** die Verweilzeit pro Heizzone der Trocknungsanlage etwa 1 bis 8 Minuten beträgt.

8. Verfahren nach mindestens einem der Ansprüche 6 bis 7, **dadurch gekennzeichnet, dass** die Trocknungsanlage in (b1) drei Heizzonen aufweist, wobei T₁. Heizzone < T₂. Heizzone >T₃. Heizzone, und T₁. Heizzone ≥ T₃. Heizzone.

9. Verfahren nach mindestens einem der Ansprüche 1 bis 5, **dadurch gekennzeichnet, dass** die Temperaturbehandlung gemäß Schritt (b2) bei einem Druck von etwa 5 bis etwa 300 mbar erfolgt.

10. Verfahren nach Anspruch 9, **dadurch gekennzeichnet, dass** die Temperaturbehandlung gemäß Schritt (b2) im Bereich von etwa 30 bis etwa 40 °C erfolgt.

11. Verfahren nach mindestens einem der Ansprüche 9 bis 10, **dadurch gekennzeichnet, dass** die Verweilzeit in der Trocknungsanlage etwa 1 bis 4 Stunden beträgt.

12. Verfahren nach einem der vorhergehenden Ansprüche, **dadurch gekennzeichnet, dass** die Konditionierung gemäß (c1) im Bereich etwa 55 bis etwa 65 °C erfolgt.

13. Verfahren nach Anspruch 12, **dadurch gekennzeichnet, dass** die Konditionierung gemäß (c1) über einen Zeitraum von etwa 30 Minuten bis 6 Stunden bei einer Temperatur > 50 °C erfolgt.

14. Verfahren nach einem der vorhergehenden Ansprüche, **dadurch gekennzeichnet, dass** die Konditionierung gemäß (c2) über einen Zeitraum von etwa 24 bis etwa 72 Stunden und Luft auf eine Temperatur von etwa 30 bis etwa 35 °C erfolgt.

15. Allulosekristallen, die einen Gehalt an Wasser von maximal 0,1Gew.-% aufweisen, wobei die Allulosekristallen mittels des Verfahrens nach einem der vorhergehenden Ansprüche hergestellt werden.
